# EUROPEAN PATENT SPECIFICATION

(11) **EP 2 722 386 B1**
(45) Date of publication and mention of the grant of the patent: **27.09.2017**
(21) Application number: 12799972.0
(22) Date of filing: 14.06.2012
(51) Int. Cl.: C12N 1/20, A61K 35/74, A61P 3/00, A61P 25/22, A61P 31/00, A61P 35/00, A61P 37/02, A61P 37/08

(54) **CRUSHED CELLS AND COMPOSITION THEREOF**
ZERKLEINERTE ZELLEN UND ZUSAMMENSETZUNG DARAUS
CELLULES BROYÉES ET COMPOSITION À BASE DE CELLES-CI

(30) Priority: 14.06.2011 JP 2011132743
(43) Date of publication of application: 23.04.2014
(73) Proprietor: SOMA Gen-Ichiro, Setagaya-ku Tokyo 158-0084 (JP); BioMedical Research Group Inc., Setagaya-ku Tokyo 158-0084 (JP)
(72) Inventor: INAGAWA, Hiroyuki, Takamatsu-shi Kagawa 761-8062 (JP); KOHCHI, Chie, Takamatsu-shi Kagawa 761-8075 (JP); SOMA, Gen-Ichiro, Setagaya-ku Tokyo 158-0084 (JP)
(74) Representative: Appelt, Christian W.
(86) International application number: PCT/JP2012/065173
(87) International publication number: WO 2012/173163

(56) References cited:
- EP-A1- 0 416 892
- EP-A1- 0 941 736
- EP-A1- 1 961 823
- WO-A1-2007/061102
- JP-A- 2004 262 773
- JP-B2- 2 526 733
- DATABASE EPODOC [Online] EUROPEAN PATENT OFFICE, THE HAGUE, NL; 27 November 1990 (1990-11-27), XP002720872, Database accession no. JP-32476090-A -& JP 2 526733 B2 (AJINOMOTO KK) 21 August 1996 (1996-08-21)
- VALENTINA TAVERNITI ET AL: "The immunomodulatory properties of probiotic microorganisms beyond their viability (ghost probiotics: proposal of paraprobiotic concept)", GENES & NUTRITION ; STUDYING THE RELATIONSHIP BETWEEN GENETICS AND NUTRITION IN THE IMPROVEMENT OF HUMAN HEALTH, vol. 6, no. 3, 16 April 2011 (2011-04-16), pages 261-274, XP019931658, SPRINGER-VERLAG, BERLIN/HEIDELBERG ISSN: 1865-3499, DOI: 10.1007/S12263-011-0218-X
- ADAMS C A: "The probiotic paradox: Live and dead cells are biological response modifiers", NUTRITION RESEARCH REVIEWS, vol. 23, no. 1, 1 June 2010 (2010-06-01), pages 37-46, XP009145190, CAMBRIDGE UNIVERSITY PRESS, CAMBRIDGE, GB ISSN: 0954-4224, DOI: 10.1017/S0954422410000090
- CHIE KOCHI ET AL.: 'Development of lipopolysaccharide material characterized by safety and enhancement of innate immunity' THE FOOD INDUSTRY 30 December 2010, pages 52 - 61, XP008168225
- HIROYUKI INAGAWA ET AL.: 'Kenko Shikosei Shokuhin Sozai to shite no Gram Inseikin Lipopolysaccharide (LPS) no Kanosei' NEW FOOD INDUSTRY vol. 51, no. 5, 2009, pages 37 - 42, XP008167957
- HIROYUKI INAGAWA ET AL.: 'New Approach of Lipopolysaccharide Derived from Dietary-Experienced Gram-Negative Bacteria (IP-PA1) Based on the Macrophage-Network' JAPANESE JOURNAL OF COMPLEMENTARY AND ALTERNATIVE MEDICINE vol. 4, no. 2, 2007, pages 79 - 90, XP008168121
- SAIBO HAKAI SHIN SEIKAGAKU KOZA 17 BISEIBUTSU JIKKENHO 1992, pages 173 - 178, XP008168821

## Description

### [Technical Field]

The present invention relates to compositions containing a microbial crushed bacterial body for use in a method of prevention of cancers or allergic diseases.

### [Background Art]

All living organisms innately have an innate immunity which is a mechanism for recognizing and eliminating foreign substances (substance not present in healthy individuals: dead cells, denatured biomolecules, invading organisms, cancer cells) in living bodies. From this aspect, when innate immunity acts healthfully, the condition is healthy, and when innate immunity does not act healthfully from any cause, the condition is unhealthy. Diseases caused by abnormal functions of innate immunity may include infection, metabolic disease, accelerated senescence, cancer, infertility, dementia, allergic disease.

The most common cause of impaired function of innate immunity is stress. Physical and even psychological stresses may inhibit innate immunity. The stress inhibits macrophage which is a key cell of innate immunity. Although the cause is unclear, one of the mechanisms results from that glucocorticoid, prostaglandin, catecholamine, which are induced by stress inhibit activation of the macrophage. Stress is considered to cause many diseases, however it is difficult to reduce stress in contemporary society, and therefore, a measure for avoiding this problem is required.

As a method for avoiding inhibition of innate immunity by stress, the inventors have focused on activation of macrophage which is a key cell of innate immunity. When activation of innate immunity is controlled using activation ability for macrophage as an indicator, the inhibition of innate immunity due to stress can be avoided, resulting in prevention of worsening infections and cancer metastasis progression (Non Patent Literature 1). In addition, the ability to eliminate foreign substances is enhanced by activating the macrophage which identifies, phagocytoses and eliminates foreign substances, and thus bacterial and viral infections can be prevented. Also, preventive effects are generated for lifestyle-related diseases caused by inflammation by activating the macrophage which reduces inflammation and repairs tissues. Furthermore, this method is effective in the prevention of metabolic disease, accelerated senescence, cancer, infertility, dementia, allergic disease.

Food materials usually utilized for activating innate immunity which have been best known so far are live bacterium known as probiotics. Probiotics are provided as live bacterium contained in yogurts, supplements or the like. The bacterial species include Escherichia coli, lactic acid bacterium, bacillus bacterium, etc. (Non Patent Literature 2) .

In the past, it had been considered that immunostimulatory effects were obtained by intake of live bacterium and then bacterial proliferation in the intestine was required, and killed bacterium could generate no or extremely low effects (Non Patent Literature 2). However, in recent years, it has been revealed that not only live bacterium but also killed bacterium has immunostimulatory effects (Non Patent Literature 3). For example, recently, killed lactic acid bacterium has been widely used as immunostimulatory food material in Japan. Both live and killed bacterium is considered to have the same effects, because components in the bacterium activate immunity as a mechanism effective even in killed bacterium (Non Patent Literature 4).

Bacteria-derived components for activating macrophage (and innate immunity) may include lipopolysaccharide, lipoteichoic acid, lipoarabinomannan, peptidoglycan, flagellin, lipoprotein, muramyldipeptide, proteoglycan, a gene including unmethylated cytosine/guanine sequence, β-glucan, etc. These bacterial components are recognized by Toll-like receptor (TLR), NOD-like receptor (Nucleotide binding oligomerization domain-like receptor: NLR), Type C lectin receptor (CLR) and the like which are included in innate immune cells such as macrophage, neutrophil, dendritic cell, natural killer cell, B-1 cell, mucosal epithelial cell (Non Patent Literature 5). As a result, the innate immune cells which incorporated them are activated. These components are recognized on the outer membrane of the cell, otherwise are incorporated in the cell and recognized by follicles (endosome, phagosome) and protein complexes (inflammasome). That is, although these bacterial components are considered to be useful as innate immunostimulatory substances, it is actually indicated that they also have infection-preventing effects, growth promoting effects and the like (Patent Literatures 1 and 2).
Patent Literature 4 describes the use of a low moleculor weight LPS obtained from Acetobacter.

However, lipopolysaccharide, lipoteichoic acid, lipoarabinomannan, peptidoglycan, flagellin, lipoprotein, muramyldipeptide, proteoglycan, a gene including unmethylated cytosine/guanine sequence and the like can be prepared by purification or synthesis from bacterium, but the cost is extremely high. The components are sold for tens of thousands yen per 1mg. Consequently, they are difficult to make commercially viable as preventive foods or health-maintenance supplements in light of cost.

On the other hand, live bacterial bodies and killed bacterial bodies can be produced at a considerably lower cost than that of purified bacterial components. However, the amount of the bacterium actually used is great. For example, killed lactic acid bacterium of as much as 150mg/kg body weight (about 1 g: 1 trillion cells per person) on a dry weight basis is used (Non Patent Literature 6). Thus, if the effects of the live bacterium and killed bacterium can be increased at a low cost, the cost can be considerably reduced.

It has been reported that the effects can be increased by physically or enzymatically crushing killed bacterial bodies obtained through treatment at 100°C for 10 minutes and subsequent centrifugation (Patent Literature 3). Even if the amount is reduced to one-fifth, equivalent effects can be obtained by crushing Bacillus subtilis (gram-positive bacterium) and Brevibacteriumlactofermentum (gram-positive bacterium), but in Streptococcus thermophilus (gram-positive bacterium), Lactobacillus acidophilus (gram-positive bacterium) and Streptomyces tanashiensis (gram-positive bacterium), the effects are reduced by about half. All of them are gram-positive bacteria, and these reports indicate that bacterial bodies and crushed bacterial bodies of Escherichia coli (gram-negative bacterium) achieve higher effects than in the case of LPS alone, but the higher effects of crushed bacterial bodies than of bacterial bodies are limited to some gram-positive bacteria.

Bacteria can be distinguished between gram-positive bacteria and gram-negative bacteria by a difference in gram stainability, and this difference in stainability results from a difference in thicknesses of cell walls (peptidoglycan layer). Gram-positive bacteria have thick cell walls (peptidoglycan layer), and those of gram-positive bacteria are thin. Thereby, in a case of a gram-positive bacterium, decolorization of pigment is inhibited due to its thick cell wall in the process of Gram's staining, and the pigment remains. Meanwhile, a peptidoglycan layer of a gram-negative bacterium is thin and decolorized, and therefore, is not gram-stained (Non Patent Literature 7). An amount of the peptidoglycan is as high as 90% on a dry weight basis in the gram-positive bacterium and far higher compared to 10% of that in the gram-negative bacterium (Non Patent Literature 8).

The peptidoglycan in the bacterial cell wall is a super high-molecular substance comprising peptides and carbohydrates, which is also a rigid substance maintaining the morphology and strength of the cell (Non Patent Literature 8) . The peptidoglycan extracted from cells and a muramyldipeptide obtained by decomposing the peptidoglycan can activate innate immune cells through a dectin-1 receptor, NOD-1, NOD-2, TLR2, on the surface of the immune cells.

On the other hand, the gram-negative bacterium has an extracellular membrane which is absent in the gram-positive bacterium and has lipopolysaccharides (LPS) on the membrane. The lipopolysaccharide has a structure in which a carbohydrate chain comprising various carbohydrates binds to a lipid called lipid A. The carbohydrate chain moiety is composed of a moiety called core polysaccharide and a moiety called O-antigen. The lipid A moiety of the lipopolysaccharide enters a lipid layer of the extracellular membrane. In relation to bioactivity of the lipopolysaccharide, cells are activated through the Toll-like receptor 4 (TLR4), MD-2 and CD14 on cell surfaces of immune cells (Non Patent Literatures 9 and 10). As seen from the above, the structures of the gram-positive bacteria and the gram-negative bacteria are completely different from each other, this may lead to a result that the gram-negative bacteria do not generate effects even by bacterial body treatment effective for the gram-positive bacteria.

In addition, the primary immune-activating substance of the gram-positive bacteria is peptidoglycan, and peptidoglycan is present in not only the gram-positive bacteria but also the gram-negative bacteria. And, the immunostimulatory ability of peptidoglycan is much lower than that of LPS. From this aspect, it is considered that the gram-negative bacteria have much higher immunostimulatory effects than those of the gram-positive bacteria. Thus, if a method for amplifying the effects of the gram-negative bacterial bodies at a low cost can be established, an immunostimulatory material that is far more efficient than the gram-positive bacteria can be manufactured.

The inventors had made intense efforts to produce a gram-negative crushed bacterial body which increases immunostimulatory effects of gram-negative bacterial bodies at a low cost, and finally, the inventors succeeded in production of a crushed bacterial body for obtaining effects equivalent to those of bacterial bodies at one-fifth to one-thirtieth of the amount.

### [Citation List]

### [Patent Literature]

[Patent Literature 1] JP 4043533 B2
[Patent Literature 2] JP 6-217712 A
[Patent Literature 3] JP 2526733 B2
Patent Literature 4] EP 1 961 823 A

### [Non Patent Literature]

[Non Patent Literature 1] Takeru Nakamoto, et al., Treatments for the activating macrophages that reduces surgical stress and postoperative mortalities from bacterial infections and tumor metastases. in vivo 21: 357-364 (2007)
[Non Patent Literature 2] R. Herich, MLevkut, Lactic acid bacteria, probiotics and immune system. Vet. Med.-Czech, 47, 169-180, 2002.
[Non Patent Literature 3] Elisa OVintini 1, and Marcela S Medina 1, Host Immunity in the protective response to nasal immunization with a pneumococcal antigen associated to live and heat-killed Lactobacillus casei. BMC Immunology, 12: 46, 2011
[Non Patent Literature 4] Adams CA, Nutrition Research Review, 2010, 23 (1), 37-46,
   or Adriana A Pedroso and Margie D Lee, Poultry Informed Professional, 114, 1-4, 2010.
[Non Patent Literature 5] Wikipedia "Toll-like receptor" in Japanese
[Non Patent Literature 6] Takamitsu T, et al., Effect of a cell preparation of Enterococcus faecalis strain EC-12 on digesta flow and recovery from constipation in a pig model and human subjects. Microbial Ecology in Health and Disease. 17, 107-113, 2005.
[Non Patent Literature 7] Wikipedia "Gram staining" in Japanese [Non Patent Literature 8] Wikipedia "Peptidoglycan" in Japanese
[Non Patent Literature 9] Wikipedia "Lipopolysaccharide" in Japanese
[Non Patent Literature 10] Wikipedia "Lipid A" in Japanese [Non Patent Literature 11] Takashi Nishizawa, et al., Homeostasis as regulated by activated macrophage. I. Lipopolysaccharide (LPS) from wheat flour: Isolation, purification and some biological activities. Chem. Pharm. Bull. 40:479-483 (1992)

### [Summary of Invention]

### [Technical Problem]

In light of the above-mentioned problems, an object of the present invention is to provide an innate immunostimulatory substance derived from a gram-negative microbial crushed bacterial body, and to provide an inexpensive and practical microbial crushed bacterial body a composition and drug thereof.

### [Solution to Problem]

The present invention relates to a composition for use in a method of prevention of cancers or allergic diseases, said composition containing a crushed bacterial body, in accordance with the appended claims.

The crushed bacterial body of the present invention is characterized in that it can be obtained by culturing a gram-negative bacterium and physically crushing a body of the gram-negative bacterium, comprises all components including immunostimulatory components in the bacterial body, and comprises LPSs having molecular weights of 20,000 or less as active ingredients.

Also, the gram-negative bacterium is desirably Escherichia coli, Serratia bacterium, Aeromonas bacterium, Rahnella bacterium, Enterobacter bacterium, Xanthomonas bacterium, Zymomonas bacterium, Pantoea bacterium or Acetobacter bacterium.

The composition of the crushed bacterial body of the present invention is characterized by a drug, food, functional food, feedstuff, pet food or veterinary drug which comprise the crushed bacterial body.

### [Advantageous Effects of Invention]

According to the present invention, the crushed bacterial body capable of inducing production of nitric oxide is effective as a health food, drug, skin-care product, bath powder, feedstuff for protecting crustaceans, shellfish, fish, poultry, farm animals and pet animals from infections, and as feed additives for growth promotion. In addition, as a form adopted for these feed additives, liquids, solid feed, pasty feed and supplements for pet or the like can be advantageously used.

### [Description of Embodiments]

Hereinafter, an embodiment for carrying out the present invention will be described in detail. As a result of intense study and research, the inventors found that a simply-processed gram-negative bacterial body showed more potent immunostimulatory ability than that of a non-crushed bacterial body, a purified LPS and peptidoglycan, a purified DNA alone, leading to completion of the invention.

After a gram-negative bacterium is cultured with usual nutrients, the bacterial body may be isolated from the culture. The species of the bacterium is not particularly restricted, if the bacteria is gram negative and contains LPS. For example, the bacterium may be Escherichia coli, Salmonella bacterium, Aeromonas bacterium, Acinetobacter bacterium, Proteus bacterium, Serratia bacterium, Bordetella pertussis, Yersinia bacterium, Neisseria bacterium, etc. Particularly, edible gram-negative bacteria, Acetobacter bacterium, Xanthomonas bacterium, Zymomonas bacterium, Pantoea bacterium, Enterobacter bacterium, etc., are desirable from a viewpoint of history as foods. In addition, the major molecular weight of the LPSs of the gram-negative bacterium that having histories as foods is 20,000 or less.

An untreated or heat-sterilized bacterial body is mechanically crushed. Desirably, the crushing treatment is non-enzymatically carried out at a low temperature. The crushing of bodies can be confirmed under a microscope, and the crushed bacterium account for 90% or more, preferably about 100%. When a high pressure is used for crushing, crushing is preferably conducted during cooling, and the cooling temperature is 20°C or lower, more preferably 4°C or lower. The method in which bacterium are frozen for crushing is preferable in light of cooling. In a case of high-pressure crushing, 400 psi or higher is preferable.

As an evaluation method for innate immune-activation, priming effect (effect induced by primary irritant) can be utilized. The priming effect is defined as an effect which itself does not induce definite action but significantly amplifies effects induced by a stimulation (secondary stimulation) in the subsequent process. In this case, the induced effects can be regarded as various biological responses. Their examples may include tumor necrosis factor (TNF) induction, interleukin (IL)-1α induction, IL-1β induction, nitric oxide induction, active oxygen induction, cancer cell injury, etc. A priming phase (state) means a state where induction is caused by the priming effect, and in this state, stress resistance, infection protection effect, cancer metastasis suppression effect and the like can be obtained (Non Patent Literature 1). For example, interferon-γ is administered to a mouse as a primary stimulation, and three hours later, a lipopolysaccharide is administered as a secondary stimulation, and the level of tumor necrosis factors in the serum after one hour is measured, indicating that the level of the tumor necrosis factors increases by about 3-10 times by the primary stimulation. In this case, when only primary stimulation is given, the level of the tumor necrosis factors in serum is within a range that it can be detected as a healthy condition. In this case, the interferon-γ is called primary stimulator (primer), and the lipopolysaccharide is called secondary stimulator (trigger). Thus we can recognize the tumor necrosis factor induced into blood by administration of the trigger as a biological response.

Similarly, the priming effect can be examined by using cells. For the cells, macrophage which is a key cell of innate immunity can be primarily used. The macrophage is a cell which is distributed evenly over the entire body. Although the macrophage functionally discriminates and eliminates foreign substances, properties of the macrophages vary by tissue involving each macrophage because of an ability to change properties to suit an environment. However, all macrophages have functions to eliminate foreign substances, and therefore, microglia in the brain, alveolar macrophage in the lungs, Kupffer cells in the liver, Langerhans cells in the skin, peritoneal macrophage, blood-derived monocyte, cells isolated from tissues such as bone marrow cells, and established cell lines can be used. For example, a primer such as interferon-γ is added to macrophage cell lines (RAW246.7, J774.1, THP-1, NR8383), peritoneal macrophage, peripheral blood monocyte, and macrophage obtained by differentiation induction of bone marrow cells, and cultured, to which OK-432 (Picibanil: hemolytic streptococcus preparation, Chugai Pharmaceutical Co., Ltd.), LPS, killed lactic acid bacterium body, etc., are then added. Then, the priming effects can be evaluated by measuring TNF, IL-1β, active oxygen, nitric oxide, etc., which were induced into a culture supernatant. Also, they can be evaluated by quantitatively measuring yields of transcribed RNAs of the tumor necrosis factor, IL-1β and inducible nitric oxide synthase which are induced into the macrophage cells. This priming ability (priming activity) is used as an indicator, thereby the priming effect can be evaluated as an indicator of innate immunostimulatory ability, even by a mixture of lipopolysaccharide, lipoteichoic acid, lipoarabinomannan, peptidoglycan, flagellin, lipoprotein, muramyldipeptide, proteoglycan and a gene including unmethylated cytosine/guanine sequence which are components of the gram-positive and negative bacteria.

### [Example 1]

As gram-negative bacteria, Escherichia coli, Pantoea bacterium (Pantoea agglomerans), Serratia bacterium (Serratia ficaria), Aeromonas bacterium (Aeromonas hydrophila), Rahnella bacterium (Rahnella aquatilis), Enterobacter bacterium (Enterobacter cloacae), Xanthomonas bacterium (Xanthomonas campestris) and Zymomonas bacterium (Zymomonas mobilis) are spread on a conventional agar medium for bacterial culture, and cultured at 37°C. As the agar medium, for example, a standard agar medium, a brain-heart infusion agar medium, can be used. One of the colonies that emerged is taken, and this can be cultured with a conventional liquid medium, for example, Trypticase soy broth and nutrient broth (Becton, Dickinson and Company), using an adequate culture flask, e.g. a 3-liter shake flask or the like. A shaking culture was conducted overnight at 37°C. After culturing, the bacterial body was precipitated by centrifugation (2000 g, 10 min.) to collect each bacterial body.

We prepared a sample of the Escherichia coli. We dispersed 1g of each bacterial body in 10ml of phosphate buffered saline (PBS) (live bacterial dispersion). In Patent Literature 3, a suspension of the cultured bacteria (live bacteria) was collected after heating (Column 5, Line 20-22: This treatment method for the bacterial body is called the "conventional form"). In this case, it is considered that some components of the bacterial bodies are lost by collecting the bacterial bodies after heating. Meanwhile, the present invention is intended to crush the bacterial bodies and to provide a crushed bacterial body which contains all components including immunostimulatory components in the bacterial bodies.

After the live bacterial dispersion was heated at 100°C for 10 minutes, the pellet was collected by a centrifuge, and resuspended in PBS to prepare a non-heat-killed bacterium (conventional heat-killed bacterial body).
- Formalin was added to the live bacterial dispersion so that the concentration of Formalin was 0.5%, and this was stored at room temperature for 1 hour for sterilization, then the pellet was collected by the centrifuge, formalin was removed, and the pellet was resuspended in PBS to prepare a non-heat-killed bacterium (non-heat-killed bacterial body).

After the live bacterial dispersion was heated by an autoclave at 100°C for 10 minutes, the heated bacterial body was crushed by homogenization (Polytron homogenizer) (homogenate bacterial body).
- After the live bacterial dispersion was heated by an autoclave at 100°C for 10 minutes, the heated bacterial body was ultrasonicated for 30 minutes to crush the bacterial bodies (ultrasonically-crushed bacterial body).

After the live bacterial dispersion was heated at 100°C for 10 minutes, the heated bacterial body was cooled at 4°C, and crushed by a'high-pressure cell crusher (2000 psi) (high-pressure-crushed bacterial body). The degree of crushing was observed under a biological microscope at 1000-fold magnification, and a state that almost all the bacterial forms were broken (above 95% crush) was confirmed.

A sample of the Pantoea bacterium was prepared. 1g of each bacterial body was dispersed in 10ml of phosphate buffered saline (PBS) (live bacterial dispersion).
- After the live bacterial dispersion was heated at 100°C for 10 minutes, the pellet was collected by a centrifuge, and resuspended in PBS to prepare a non-heat-killed bacterium (conventional heat-killed bacterial body).
- Formalin was added to the live bacterial dispersion so that the concentration of Formalin was 0.5%, which was stored at room temperature for 1 hour for sterilization, and then the pellet was collected by the centrifuge, formalin was removed, and the pellet was resuspended in PBS to prepare a non-heat-killed bacterium (non-heat-killed bacterial body).

After the live bacterial dispersion was heated at 100°C for 10 minutes, the pellet was collected by a centrifuge, resuspended in PBS, cooled to 4°C, and crushed by a high-pressure crusher (Contact Systems, Inc, 2000 psi) (conventional pre-treated high-pressure-crushed bacterial body).
- After the live bacterial dispersion was heated by an autoclave at 100°C for 10 minutes, the pellet was collected by the centrifuge, then resuspended in PBS, and homogenized (Polytron homogenizer) (conventional pre-treated homogenate bacterial body).

After the live bacterial dispersion was heated at 100°C for 10 minutes, the heated bacterial body was cooled at 4°C, and crushed by a high-pressure cell crusher (2000 to 20000 psi) (high-pressure-crushed bacterial body). The degree of crushing was observed under a biological microscope at 1000-fold magnification, and we confirmed that a state that almost all the bacterial forms were broken (above 95% crushed) .
- After the live bacterial dispersion was heated by an autoclave at 100°C for 10 minutes, the heated bacterial body was crushed by homogenization (Polytron homogenizer) (homogenate bacterial body).

For each treated bacterial body, it was confirmed by an agar medium that there is no grown cell (cell was killed). Note that, the "killed bacterium" herein means that the bacterium is dead but the structure and morphology (e.g. coccus is spherical, and bacillus is columnar) are essentially maintained, and the bacterium broken by the present invention means a state that the morphology of the bacterial body is not maintained.

Purification of the LPS: We carried out the extractive purification of LPS on the basis of a conventional method (1992CPB-I).

For Serratia bacterium, Aeromonas bacterium, Rahnella bacterium, Enterobacter bacterium, Xanthomonas bacterium and Zymomonas bacterium, the following samples were prepared. We dispersed 1g of each bacterial body in 10ml of phosphate buffered saline (PBS) (live bacterial dispersion).
- After the live bacterial dispersion was heated at 100°C for 10 minutes, the pellet was collected by a centrifuge, and resuspended in PBS to prepare a non-heat-killed bacterium (conventional heat-killed bacterial body).
- After the live bacterial dispersion was heated at 100°C for 10 minutes, the heated bacterial body was cooled at4°C, and crushed by a high-pressure cell crusher (2000-20000 psi) (high-pressure-crushed bacterial body). The degree of crushing was observed under a biological microscope at 1000-fold magnification, and a state that almost all the bacterial forms were broken (above 95% crushed) was confirmed.

For each treated bacterial body, it was confirmed by an agar medium that there is no grown cell (cell was killed).

Acetobacter bacterium (Acetobacter aceti, Gluconobacter cerinus) was spread on a conventional Acetobacter selective medium, for example, the modified Aggie's agar medium (2% of glucose, 1% of glycerol, 2% of ethanol, 1.5% of yeast extract, 1% of polypeptone, and agar), and cultured at 30°C. We take one of the colonies that emerged in the medium, and culturing could be conducted in the modified Nodai agar medium or a nutrient broth using an appropriate culture flask, e.g. a 3-liter shake flask or the like. A shaking culture was conducted at 30°C for 3 days. After culturing, the bacterial body was precipitated by centrifugation (2000g, 10 min.) to collect Acetobacter bacteria. The number of bacterial bodies was measured by a counting chamber. 1g of Acetobacter bacterial body was dispersed in 10ml of phosphate buffered saline (PBS) (bacterial dispersion), heated by an autoclave at 120°C for 20 minutes, then crushed by homogenizer, by ultrasonic for 30 minutes, and by a high-pressure cell crusher (15000 psi) respectively. 9ml of PBS was added to the high-pressure-crushed bacterial body. In addition, phenol was added to the bacterial dispersion so that it was 0.1% or formalin was added so that it was 0.5%, which was stored at room temperature for 1 hour to kill the bacteria, then the pellet was collected by a centrifuge, the phenol or formalin was removed, and the pellet was resuspended in PBS to prepare a non-heated killed Acetobacter bacterial body. In addition, as a conventional method, the bacterial dispersion was heated at 100°C for 10 minutes, then the pellet was collected by a centrifuge, a killed Acetobacter bacterial body dispersed in water was prepared, and the bacterium was ultrasonicated for 30 minutes. For each treated Acetobacter bacterium, it was confirmed by the modified Aggie' s agar medium that the Acetobacter bacterium was killed.

1g of Acetobacter bacterial body was dispersed in 10ml of phosphate buffered saline (PBS), heated at 100°C for 10 minutes, then the heated bacterial body was cooled at 4°C, and crushed by a high-pressure cell crusher (20,000 psi) (high-pressure-crushed bacterial body). The degree of crushing was observed under a biological microscope, and a state that almost all the bacterial forms were broken (above 95% crushed) was confirmed.

For the bacterial body, it was confirmed by the modified Aggie's agar medium that there is no grown bacterium (bacterium was killed).

### [Example 2]

### Experiments for activations of Escherichia coli and macrophage cell: Evaluation of capacity to produce nitric oxide

We evaluated an activation ability for the macrophage for its capacity to produce nitric oxide by each treatment of Escherichia coli and Pantoea bacterium bodies. When the macrophage is stimulated by lipopolysaccharide, lipoteichoic acid, lipoarabinomannan, peptidoglycan, flagellin, lipoprotein, muramyldipeptide, proteoglycan, a gene including unmethylated cytosine/guanine sequence, etc., a nitric oxide synthase is induced in macrophage, resulting in production of nitric oxide. Since nitric oxide is one of activating gases and has cytotoxic activity to bacteria, viruses and cancer cells, it works as a molecule responsible for elimination of foreign substances in the activated macrophage. Since nitric oxide is unstable and changes into nitrite, measurement was carried out for nitrite.

A conventional heat-killed bacterial body, a homogenate bacterial body, an ultrasonically-crushed bacterial body and a high-pressure-crushed bacterial body (2000 psi) of Escherichia coli were individually prepared so that their bacterial body weights were 20ng/ml, 200ng/ml, 2µg/ml and 20µg/ml. In a CO2 incubator, RAW246.7 cell was cultured in an RPMI1640 medium to which 10% of fetal bovine serum was added. A culture was started with an initial concentration of 50,000 cells/ml and cells grew to about 2,000,000/ml, and at this time, the broth was readjusted to the initial concentration and subcultured. For the experiment, RAW cells were put into each well (96-well culture plate) at 50,000 cells/0.1ml/well, to which a 0.1ml/well of the prepared sample was added with final concentrations of 10ng/ml, 100ng/ml, 1µg/ml and 10µg/ml, cultured for 24. hours, and a culture supernatant was collected.

For the activation ability for macrophage, a concentration of nitrite that is a nitric oxide metabolite to be induced in this test was measured by Griess reagent.

In a case that the amount of nitrite is 10µM in the culture supernatant 24 hours after stimulation of macrophage (RAW246.7 cell) at 1µg/ml, the activation ability of the substance for macrophage is designated as 1 unit (unit/ml). For the activation ability of an arbitrary sample for macrophage, a concentration of nitrite induced by the arbitrary diluted sample is measured, and the sample concentration (logarithm) and the nitrite concentration are plotted on a semilogarithmic graph to estimate the sample concentration which induces 10µM of nitrite. A multiple number for adjusting this concentration to 1µg/ml is determined and is designated as a macrophage activation unit. For example, when a sample has a concentration of 100ng/ml enough to induce 10µM of nitrite, a calculation formula 1µg/ml ÷ 100ng/ml = 10 is given, and hence this sample is to have 10 units of activation ability for macrophage.

### Results

We show concentrations of the produced nitrite in each concentration of each treated Escherichia coli sample in Table 1. The concentration of the unstimulated RAW246.7 cell in the culture supernatant was 2.8-3.1µM which was a detection limit of nitrite. When bacteria concentrations enough to induce 10µM of nitrite were determined from the nitrite concentrations at each concentration of each treated Escherichia coli body by plotting the concentrations on the semilogarithmic graph, the concentration of the killed Escherichia coli (conventional heat-killed bacterial body) was 500ng/ml and 2 units by a conventional method, meanwhile, the homogenate bacterial body showed 16ng/ml (62.5 units), the ultrasonically-crushed bacterial body showed 58ng/ml (17.2 units), and the high-pressure-crushed bacterial body showed 200ng/ml (5 units). That is, this result reveals that activation ability for macrophage is increased by as much as 2.5-31 times by treatment even supposing that they are derived from the same Escherichia coli body.
[Table 1]

**Table 1 Concentrations of the nitrite produced from macrophage by the treated samples of Escherichia coli body**

| | 0ng/ml | 10ng/ml | 100ng/ml | 1µg/ml | unit |
|---|---|---|---|---|---|
| Killed Escherichia coli body in conventional method (conventional heat-killed bacterial body) | 3.0 | 3.4 | 5.6 | 12.1 | 2 |
| Heat-homogenized bacterial body (homogenate bacterial body) | 2.8 | 8.5 | 13.3 | 14.1 | 62.5 |
| Ultrasonicated bacterial body (ultrasonically-crushed bacterial body) | 3.1 | 7.0 | 11.1 | 13.7 | 17.2 |
| High-pressure-crushed cell bacterial body (high-pressure-crushe d bacterial body) | 2.9 | 4.1 | 8.3 | 12.8 | 5 |

Using Pantoea bacterium, a conventional heated killed bacterial body, a non-heat-killed bacterial body, a conventional pre-treated high-pressure-crushed bacterial body, a conventional pre-treated homogenate bacterial body, a high-pressure-crushed bacterial body (20000 psi), a homogenate bacterial body were prepared, and individually diluted so that their bacterial body weights were 2ng/ml, 20ng/ml, 200ng/ml, 2µg/ml and 20µg/ml. In a CO₂ incubator, a RAW246.7 cell was cultured in an RPMI1640 medium to which 10% of fetal bovine serum was added. The culture was started with an initial concentration of 50,000 cells/ml and cells grew to about 2,000,000/ml, and at this time, the broth was readjusted to the initial concentration and subcultured. For the experiment, we put RAW cells into each well (96-well culture plate) at 50,000 cells/0.1ml/well, added 0.1ml/well of the prepared sample with final concentrations of 1ng/ml, 10ng/ml, 100ng/ml, 1µg/ml and 10µg/ml, cultured for 24 hours, and collected a culture supernatant.

### Results

The nitrite concentration in the culture supernatant of the unstimulated RAW246.7 cell is designated as 0µM of the nitrite yield, and each concentration of produced nitrite in each treated Pantoea bacterium sample was shown in Table 2. The bacteria concentrations enough to induce 10µM of nitrite in each sample were plotted on the semilogarithmic graph, and the number of units in each sample was calculated and shown in Table 2. The conventional heat-killed bacterial body showed 400ng/ml and 2.5 units, meanwhile, the conventional pre-treated high-pressure-crushed bacterial body and the conventional pre-treated homogenate bacterial body showed 300ng/ml (3.3 units), the non-heat-killed bacterial body showed 185ng/ml (5.4 units), the homogenate bacterial body showed 44ng/ml (23 units), and the high-pressure-crushed bacterial body showed 12ng/ml (83 units). A relative ratio can be calculated from this result on the basis of the conventional heat-killed bacterial body, and thus we summarised them in table 2. The case of conventional treatment, the ability was only improved by about 1. 3 times (an equivalent effect can be obtained at a 1-in-1.3 concentration). On the other hand, when the bacterial bodies were homogenized or high-pressure-crushed instead of the conventional treatment, the abilities could be remarkably improved by 9.2-33.2 times.
[Table 2]

**Table 2 Concentrations of the nitrite produced from macrophaqe by the treated samples of Pantoea bacterial body**

| | 0ng/ml | 1ng/ml | 10ng/ml | 100ng/ml | 1ug/ml |
|---|---|---|---|---|---|
| Conventional heat-killed bacterial body | 0 | 0 | 0 | 1.0 | 16 |
| Non-heat-killed bacterial body | 0 | 0 | 0 | 5.8 | 24.4 |
| Conventional pre-treated high-pressure-crus hed bacterial body | 0 | 0 | 0 | 3.3 | 17.2 |
| Conventional pre-treated homogenate bacterial body | 0 | 0 | 0 | 2.6 | 18 |
| High-pressure-crus hed bacterial body | 0 | 0 | 8.8 | 23.4 | 26.7 |
| Homogenate bacterial body | 0 | 0 | 1.0 | 16.1 | 25.3 |

[Table 3]

**Table 3 Evaluation for activation of macrophage by each treated sample of Pantoea bacterium**

| | (Estimated) Sample concentration required for inducing 10µM | Unit | Relative ratio |
|---|---|---|---|
| Conventional heat-killed bacterial body | 400ng/ml | 2.5 | 1 |
| Non-heat-killed bacterial body | 185ng/ml | 5.4 | 2.2 |
| Conventional pre-treated high-pressure-crushed bacterial body | 300ng/ml | 3.3 | 1.3 |
| Conventional pre-treated homogenate bacterial body | 300ng/ml | 3.3 | 1.3 |
| High-pressure-crushed bacterial body | 12ng/ml | 83 | 33.2 |
| Homogenate bacterial body | 44ng/ml | 23 | 9.2 |

### [Example 3]

The high-pressure-crushing treatment of Pantoea bacterium that showed the highest effects was conducted with crushing pressures of 2000 psi, 5000 psi, 10000 psi and 20.000 psi, and the ability to produce nitric oxide from RAW246.7 cell was evaluated in the same way as the above example. The relative ratio was calculated on the basis of the conventional heat-killed bacterial body, and summarized in Table 4. On the basis of the conventional heat-killed bacterial body, the ability was improved by 8.5-21.2 times at 2000-20000 psi in high-pressure crushing, but the effects on the pressure were slightly changed, and all pressures within this range could be used.
[Table 4]

**Table 4 Evaluation for activation of macrophage by each high-pressure-crushed sample of Pantoea bacterium**

| | Unit | Relative ratio |
|---|---|---|
| Conventional heat-killed bacterial body | 4.5 | 1 |
| High-pressure-crushed bacterial body 2000 psi | 38.5 | 8.6 |
| High-pressure-crushed bacterial body 5000 psi | 74.0 | 16.4 |
| High-pressure-crushed bacterial body 10000 psi | 95.2 | 21.2 |
| High-pressure-crushed bacterial body 20000 psi | 60.6 | 13.5 |

### [Example 4]

The activation abilities of the high-pressure-crushed bacterial bodies for macrophage were evaluated using Escherichia coli, Serratia bacterium, Aeromonas bacterium, Rahnella bacterium, Enterobacter bacterium, Xanthomonas bacterium, Zymomonas bacterium and Acetobacter bacterium as gram-negative bacteria other than Pantoea bacterium.

Conventional heat-killed bacterial bodies and high-pressure-crushed bacterial bodies were respectively prepared with Escherichia coli, Serratia bacterium, Aeromonas bacterium, Rahnella bacterium, Enterobacter bacterium, Xanthomonas bacterium, Zymomonas bacterium and Acetobacter bacterium prepared in Example 1. Each sample was prepared so that their bacterial body weights were 2ng/ml, 20ng/ml, 200ng/ml and 2µg/ml. However, only Acetobacter bacterium was prepared so that its bacterial body weight was 2µg/ml, 20µg/ml, 200µg/ml and 2mg/ml. In a CO₂ incubator, a RAW246.7 cell was cultured in an RPMI1640 medium where 10% of fetal bovine serum was added. Culture was started with an initial concentration of 50,000 cells/ml and cells grew to about 2,000,000/ml, and at this time, the broth was readjusted to the initial concentration and subcultured. For the experiment, RAW cells were put into each well (96-well culture plate) at 50,000 cells/0.1ml/well, to which a 0.1ml/well of the prepared sample was added with final concentrations of 1ng/ml, 10ng/ml, 100ng/ml, 1µg/ml and 10µg/ml (in Acetobacter bacterium, 1µg/ml, 10µg/ml, 100µg/ml and 1mg/ml), cultured for 24 hours, and a culture supernatant was collected.

Like Example 2, the nitrite in the culture supernatant of the unstimulated RAW246.7 cell was designated as 0µM of the nitrite yield, and from each concentration of produced nitrite in each treated gram-negative bacterium sample, the bacteria concentrations enough to induce 10µM of nitrite in each sample were plotted on the semilogarithmic graph, and the number of units in each sample was calculated and shown in Table 5. All of Escherichia coli, Serratia bacterium, Aeromonas bacterium, Rahnella bacterium, Enterobacter bacterium, Xanthomonas bacterium, Zymomonas bacterium and Acetobacter bacterium showed remarkable effects 5.9-16.9 times higher than those of the conventional heat-killed bacterial body, by the treatment with the bacterial bodies of the present invention.
[Table 5]

**Table 5 Evaluation for activation of macrophage by each high-pressure-crushed sample of each gram-negative bacterial body**

| | Unit | Relative ratio |
|---|---|---|
| Conventional heat-killed body of Escherichia coli | 3.45 | 1 |
| High-pressure-crushed body of Escherichia coli | 20.8 | 6.0 |
| Conventional heat-killed body of Serratia bacterium | 4.55 | 1 |
| High-pressure-crushed body of Serratia bacterium | 76.9 | 16.9 |
| Conventional heat-killed body of Aeromonas bacterium | 6.25 | 1 |
| High-pressure-crushed body of Aeromonas bacterium | 95.2 | 15.2 |
| Conventional heat-killed body of Rahnella bacterium | 6.25 | 1 |
| High-pressure-crushed body of Rahnella bacterium | 66.7 | 10.7 |
| Conventional heat-killed body of Enterobacter bacterium | 3.03 | 1 |
| High-pressure-crushed body of Enterobacter bacterium | 20 | 6.6 |
| Conventional heat-killed body of Xanthomonas bacterium | 2.56 | 1 |
| High-pressure-crushed body of Xanthomonas bacterium | 27.8 | 10.9 |
| Conventional heat-killed body of Zymomonas bacterium | 1.85 | 1 |
| High-pressure-crushed body of Zymomonas bacterium | 20.8 | 11.2 |
| Conventional heat-killed body of Acetobacter bacterium | 0.017 | 1 |
| High-pressure-crushed body of Acetobacter bacterium | 0.1 | 5.9 |

### [Example 5]

### Measurement of priming effects using mice

For an experimental method for induction and dose dependence of priming effects in TNF production by intravenous administration of Escherichia coli bodies in mice, we used a method that was established by us. As a negative control, saline was administered. As a conventional method, the conventional heat-killed bacterial body was used. As test articles, subjects for heating and testing (killed bacterial body, autoclaved bacterial body, ultrasonicated bacterial body, high-pressure-crushed bacterial body of Escherichia coli) were used, and three C3H/He mice were used in each dose group for the test.

As a trigger, OK-432 (Picibanil; Chugai Pharmaceutical Co., Ltd.) was used. OK-432 was added to saline for injection and suspended so that the final concentration was 5KE/ml (1KE equals 0.1mg). Each test solution to be used as a primer (priming inducer) and saline in a control group were intravenously administered with doses of 0.2ml. Three hours after administration, OK-432 suspension as the trigger (TNF inducer) was intravenously administered at 1KE/0.2m1 per one mouse. Two hours later, blood was collected, heated at 37°C for 30 minutes, and then a serum was obtained by centrifugation. The serum TNF level was measured using a commercial ELISA kit (Biolegend Inc.).

For priming effects, one-half of an amplification factor of TNF induced by 1 KE of OK-432 that was amplified by 0.2µg of interferon-γ (Nippon Roche Ltd.) was designated as a standard. Any sample is adjusted to a plurality of concentrations, these are intravenously administered to mice as primers, and 3 hours later, an amplification factor of the TNF induced by OK-432 alone is determined from the TNF induced by OK-432 administered as a trigger. The degree of the priming effect (priming activity) of any sample is determined according to the following method. A concentration of any sample (logarithm) and an amplification factor of the TNF are plotted on a semilogarithmic graph to estimate the minimum sample concentration that provides one-half of the factors of the TNF induced by interferon-γ. A factor for adjusting this sample concentration to 0.1µg is determined, and designated as a priming activity unit. For example, if 10ng (0.01µg) of a sample is at one-half concentration of the TNF factor at which the TNF can be induced by 0.1µg of interferon-γ, a calculation formula 0.1µg ÷ 10ng = 10 is given, and this sample is to have 10 units of priming ability.

### Results

The serum TNF levels induced by interferon-γ and OK-432 are shown in Table 6. The serum TNF level in a case that 1 KE of OK-432 alone was administered was 125pg/ml. When 0.1µg of interferon-γ was intravenously administered to a mouse and then OK-432 was administered, the TNF level was 950pg/ml. Consequently, an amplification factor of the TNF by 0.1µg of interferon-γ was 950 ÷ 125 = 7.6 (fold). This value was multiplied by 1/2 to obtain the value of 3. 6, and concentrations of each treated bacterial sample which provide the minimum concentration giving a 3.6-fold amplification factor of the TNF were researched. The results of the doses of each treated bacterial sample and the serum TNF levels induced by OK-432 are shown in Table 7. In order to quantitatively indicate that the treatment methods enhance the activation ability compared to the conventional heat-killed bacterial bodies, relative values in each sample when the ability to induce the TNF (number of units) in the conventional heat-killed bacterial body is 1 are also shown in Table 7. The conventional pre-treated high-pressure-crushed bacterial body showed 34ng (2.9 units), the conventionally-crushed killed bacteria of Escherichia coli (ultrasonicated bacterial body) showed 27ng (3.7 units), the high-pressure-crushed bacterial body showed 0.80ng (125 units), the ultrasonicated bacterial body showed 2.1ng (48 units), and the homogenate bacterial body showed 6.5ng (15 units). That is, this result reveals that the conventional treatment method shows 1.3-fold effects without a difference, meanwhile, the treatment method developed in the present invention shows as much as 5 to 43-fold activation effects for macrophage, even supposing that they are derived from the same Escherichia coli body.
[Table 6]

**Table 6 Serum TNF level induced by administration of Interferon-γ (primer) and OK-432 (trigger) to mice**

| | TNF | Relative ratio |
|---|---|---|
| Saline | 5pg/ml or less | - |
| Interferon-γ | 5pg/ml or less | - |
| OK-432 (1KE) | 125pg/ml | 1 |
| Interferon-γ+ OK-432 | 950pg/ml | 7.6 |

[Table 7]

**Table 7 Serum TNF level induced by administration of each sample (primer) and OK-432 (trigger) to mice**

| | 1ng | 10ng | 100ng | 1µg | Unit | Relative value |
|---|---|---|---|---|---|---|
| Conventional heat-killed bacterial body | ND | 163pg/ml | 925pg/ml | 963pg/ml | 2.9 | 1 |
| Conventional pre- treated high-pressure-crushed bacterial body | ND | 225pg/ml | 875pg/ml | 1200pg/ml | 3.7 | 1.3 |
| High-pressure-crushed bacterial body | 525pg/ml | 1100pg/ml | 1200pg/ml | ND | 125 | 43.1 |
| Ultrasonicated bacterial body | 131pg/ml | 1238pg/ml | 1413pg/ml | ND | 48 | 16.6 |
| Homogenate bacterial body | 94pg/ml | 613pg/ml | 1219pg/ml | ND | 15 | 5.2 |

| | | | | | | |
|---|---|---|---|---|---|---|
| ND: not done, the serum TNF level induced by only OK-432 is 125pg/ml | | | | | | |

## Claims

1. A composition of crushed bacterial body for use in a method of prevention of cancers or allergic diseases, said composition containing a crushed bacterial body which can be obtained by culturing a gram-negative bacterium and physically crushing a heat-sterilized bacterial body of the gram-negative bacterium, and which comprises all components including immunostimulatory components in the heat-sterilized bacterial body, and comprises LPSs, wherein the LPSs have molecular weights of 20, 000 or less, as active ingredients, said composition being selected from a Pharmaceutical, food, functional food, feedstuff, pet food or veterinary drug.

2. The composition for use according to claim 1, wherein the gram-negative bacterium is Escherichia coli, Serratia bacterium, Aeromonas bacterium, Rahnella bacterium, Enterobacter bacterium, Xanthomonas bacterium, Zymomonas bacterium, Pantoea bacterium or Acetobacter bacterium.

## Patentansprüche

1. Zusammensetzung aus zerkleinertem Bakterienkörper zur Verwendung in einem Verfahren der Prävention von Krebserkrankungen oder allergischen Erkrankungen, wobei die Zusammensetzung einen zerkleinerten Bakterienkörper enthält, der durch Kultivierung eines gramnegativen Bakteriums und physikalischer Zerkleinerung eines hitzesterilisierten Bakterienkörpers des gramnegativen Bakteriums gewonnen werden kann, und wobei die Zusammensetzung alle Bestandteile, einschließlich immunstimulatorischer Bestandteile in dem hitzesterilisierten Bakterienkörper enthält und LPS enthält, wobei die LPS Molekulargewichte von 20 000 oder weniger haben, als Wirkstoffe, wobei die Zusammensetzung ausgewählt ist aus Pharmazeutika, Nahrungsmitteln, funktionellen Lebensmitteln, Futtermitteln, Heimtiernahrung oder Tierarzneimitteln.

2. Zusammensetzung zur Verwendung nach Anspruch 1, wobei das gramnegative Bakterium Escherichia coli, ein Serratia Bakterium, ein Aeromonas Bakterium, ein Rahnella Bakterium, ein Enterobacter Bakterium, ein Xanthomonas Bakterium, ein Zymomonas Bakterium, ein Pantoea Bakterium oder ein Acetobacter Bakterium ist.

## Revendications

1. Composition de corps bactérien broyé destinée à être utilisée dans un procédé de prévention des cancers ou d'affections allergiques, ladite composition contenant un corps bactérien broyé pouvant être obtenu par mise en culture d'une bactérie Gram négatif et par broyage physique d'un corps bactérien stérilisé à chaud de la bactérie Gram négatif, et qui comprend tous les constituants y compris des constituants immunostimulants dans le corps bactérien stérilisé à chaud, et comprend des LPS, dans laquelle les LPS présentent des poids moléculaires inférieurs ou égaux à 20 000, en tant que principes actifs, ladite composition étant choisie parmi un produit pharmaceutique, un aliment, un aliment fonctionnel, un aliment pour animaux, un aliment pour animal domestique ou un médicament vétérinaire.

2. Composition destinée à être utilisée selon la revendication 1, dans laquelle la bactérie Gram négatif est Escherichia coli, une bactérie Serratia, une bactérie Aeromonas, une bactérie Rahnella, une bactérie Enterobacter, une bactérie Xanthomonas, une bactérie Zymomonas, une bactérie Pantoea ou une bactérie Acetobacter.
